Europäisches Patentamt

European Patent Office

Office européen des brevets

(11) Publication number: **0 432 952 A1**

# EUROPEAN PATENT APPLICATION

(21) Application number: 90313098.7

(22) Date of filing: 03.12.90

(51) Int. Cl.⁵: **C12N 5/00**

(30) Priority: 07.12.89 JP 318439/89

(43) Date of publication of application:
19.06.91 Bulletin 91/25

(84) Designated Contracting States:
DE GB SE

(71) Applicant: SNOW BRAND MILK PRODUCTS &
CO., LTD.
1-1, Naebo-cho 6-chome Higashi-ku
Sapporo-shi Hokkaido 065(JP)

(72) Inventor: Shinmoto, Hiroshi
Sanraize 109 1-3-61, Asahicho
Kawagoe-shi, Saitama-ken(JP)
Inventor: Dousako, Shun-ichi
15-39-616, Kitaurawa 5-chome
Urawa-shi, Saitama-ken(JP)

(74) Representative: Davies, Jonathan Mark et al
Reddie & Grose 16 Theobalds Road
London WC1X 8PL(GB)

(54) Serum-free culture medium.

(57) The novel serum-free culture medium containing inorganic iron compound as a growth factor for culture of animal cells, which is easy to purify culture products from culture broth.

## SERUM-FREE CULTURE MEDIUM

The present invention relates to the serum-free culture medium used for the culture of animal cells.

In producing materials by using animal cells, the selection of the culture medium is very important for culturing cells, because the production cost of their products depends upon the price of culture medium and refining cost of the products.

There has been heretofore used a culture medium containing about 10 % of animal serum added to the basal synthetic culture medium for culturing animal cells. However, the addition of serum raised the cost of the culture medium. Not only that, but the refining cost of the product would be likewise raised by the enriched protein contained in the sera.

Thus, there has been developed a serum-free culture medium containing no sera. The serum-free culture medium has come to be in general use at present for producing materials.

There are added various cell growth factors to the serum-free culture medium. Representative examples thereof are insulin, ethanol amine, sodium selenite, transferrin, albumin, lipoprotein, cytokine, etc. Those which may raise the cost of culture medium and the purifying cost of products among them are protein growth factors. It is, therefore, indispensable to develop less protein, eventually non-protein, serum-free culture medium. The one which is comparatively expensive is transferrin among the growth factors to be added to the serum-free culture medium

Transferrin is considered to play a part as a growth factor which efficiently supplies cells with iron ions. ( Newest Medical Science, Vol 40, "Growth Promotion by Transferrin" by Kimura and Ozawa, pages 554-559, 1985, and "Cell Growth Factor", pages 152-155, compiled by Japan Association of Tissue Culture Science, 1980) The following recycling model of transferrin is asserted by Kogo , et al.

Iron-combined type of transferrin is taken into cells by endocytosis. After iron ions are isolated within cells, transferrin is again secreted outside of the cells. ( "Anemia by Iron Membrane Permeability Troubles" by Niitsu and Kogo, Separate Volume 3, Course of Medical Science, Medical Topics, page 124, 1988). The above assertion suggests that transferrin continues to supply cells with iron ions repeatedly.

On the contrary, Mether and Sato have reported that, in the serum-free culture medium containing F12 as its basal synthetic culture medium, the growth promotion by transferrin was replaced by highly concentrated iron ions. ( J.P. Mether and G.H. Sato, Experiment Cell Research, Vol 120, pages 191-200, 1979)

Further, Kovar and Franek report that, in the serum-free culture medium for mouse hybridoma with RPMI 1640 as the basal synthetic culture medium, the addition of highly concentrated 500 $\mu$M of ferric citrate makes it possible to culture the hybridoma in non-protein serum-free culture. ( Biotechnology Letters, Vol 9, pages 259-264, 1987).

However, it is known that the highly concentrated iron ions give cells troubles. It has been desired that there should be developed a serum-free culture medium capable of efficiently supplying them with iron at lower concentrations.

The present invention can provide a serum-free culture medium having therein added iron compound which may be efficiently utilized by animal cells. More particularly, the present invention can provide a serum-free culture medium having therein added iron compound which is comparatively simple in its structure. Further, the present invention can provide a serum-free culture medium which is capable of readily purifying cell products. Still further, the present invention can provide a serum-free culture medium which would be extensively used for animal cells. Further, the present invention can provide a serum-free culture medium which may be economically advantageous and be capable of mass production for industry.

The present invention has been attained by providing a serum-free culture medium with inorganic iron compound added to the serum-free culture medium as a growth factor. Namely, the inventors have discovered that a serum-free culture medium with inorganic iron compound added to the basal synthetic culture medium as a growth factor has efficiently grown animal cells, made the purification of their products easier and had extensive uses, and completed the present invention. Furthermore, the serum-free culture medium of the present invention may be readily prepared at a lower cost.

The basal synthetic culture medium of the present invention is the culture medium the principal ingredients of which are amino acid, inorganic salts, saccharide. It can maintain the basal metabolism of cells. Be that as it may, it is possible to provide for serum-free lower protein, or absolutely no protein, culture medium which is capable of culturing animal cells, through the combination of the basal synthetic culture medium ERDF (enriched RDF) with inorganic iron compound, for a prolonged period. When ERDF is used as the basal synthetic culture medium, exceedingly greater advantages are obtained, compared with other basal synthetic culture medium.

The ERDF is the conventional basal synthetic culture medium for serum-free cultivation. This has been developed by Murakami, et al. with an aim at the the growth of mouse myeloma NSl. (Nippon Nôgeikagaku Kaishi, pages 575-583, 1984) The ERDF is a mixture, as the base, of RPMI 1640, Dulbeccos Modified Minimum Essential Medium (DMEM)and Ham's F12 at the ratio of 2:1:1. (This medium shall be called RDF.)

The concentration of amino acid is trebly raised and that of glucose, doubly raised. The concentration of vitamins is also increased. Further, the osmotic pressure of the culture medium is adjusted to 290m0sm, 5 % higher than that of the normal culture medium.

The composition thereof is shown in Table 1.

Table 1

Composition  E — RDF                                    Composition  E-RDF

| NaCl | 110.0 mM | Tryptophan | 0.09 |
| KCl | 5.0 | Tyrosine | 0.48 |
| MgSO$_4$ | 0.55 | Valine | 0.93 |
| MgCl$_2$·6H$_2$O | — | | |
| Ca(NO$_3$)$_2$·4H$_2$O | — | p-Amino benzoic acid | $3.7 \times 10^{-3}$ |
| CaCl$_2$·2H$_2$O | 0.74 | Biotin | $4.1 \times 10^{-4}$ |
| NaH$_2$PO$_4$·2H$_2$O | — | Pantothenate·Ca | $2.6 \times 10^{-3}$ |
| Na$_2$HPO$_4$·12H$_2$O | 3.41 | Cholin chloride | $8.8 \times 10^{-2}$ |
| CuSO$_4$·5H$_2$O | $3.0 \times 10^{-4}$ | Folic acid | $4.1 \times 10^{-3}$ |
| FeSO$_4$·7H$_2$O | $8.0 \times 10^{-4}$ | Inositol | $2.6 \times 10^{-1}$ |
| Fe(NO$_3$)$_3$·9H$_2$O | — | Niacinamide | $1.2 \times 10^{-2}$ |
| ZnSO$_4$·7H$_2$O | $8.0 \times 10^{-4}$ | Pyridoxal·HCl | $4.9 \times 10^{-3}$ |
| | | Pyridoxine·HCl | $2.5 \times 10^{-3}$ |
| Alanine | 0.075 | Riboflavine | $5.6 \times 10^{-1}$ |
| Arginine·HCl | 2.76 | Thiamin·HCl | $4.7 \times 10^{-3}$ |
| Asparagine·H$_2$O | 0.63 | Vitamin B$_{12}$ | $2.5 \times 10$ |
| Aspartic acid | 0.30 | Lipoic acid | $2.5 \times 10^{-4}$ |
| Cysteine·HCl·H$_2$O | 0.60 | Glutathione | $1.6 \times 10^{-3}$ |
| Cystine | — | | |
| Glutamic acid | 0.27 | Glucose | 19.00 |
| Glutamine | 6.84 | Hypoxanthine | $7.5 \times 10^{-3}$ |
| Glycine | 0.57 | Putrescine·2H$_2$O | $2.5 \times 10^{-4}$ |
| Histidine·HCl·H$_2$O | 0.36 | Pyruvate·Na | 1.0 |
| Hydroxyproline | 0.24 | Thymidine | $7.5 \times 10^{-3}$ |
| Isoleucine | 1.20 | Linoleic acid | $7.5 \times 10^{-5}$ |
| Leucine | 1.26 | | |
| Lysine·HCl | 1.08 | Phenol red | 5.0 mg/ℓ |
| Methionine | 0.33 | HEPES | 1.19 g/ℓ |
| Phenylalanine | 0.45 | NaHCO$_3$ | 12.50 mM |
| Proline | 0.48 | Streptomycin | 0.1 g/ℓ |
| Serine | 0.81 | Penicillin G | $10^5$ U/ℓ |
| Threonine | 0.93 | | — |

The requirements for the ERDF of the present invention are not so strict as as described above. As long as the object and effect thereof are satisfied, it is permitted to make the deletion, addition or alteration of a part of the ingredients or the alteration of the amount thereof used. Further, there may be added insulin (I), transferrin (T), ethanol amine (E) and selenium (S) (ITES) to the ERDF. This is called ITES-ERDF. This culture medium is extensively used for culturing mouse hybridoma or human hybridoma But the ITES-ERDF is not used for the present invention, because its protein concentration is high (25μg/ml).

0.8 μM of iron sulfide is included in the ERDF.

The iron concentration is the same as that of the mixed culture medium, RPMI 1640 : DMEM : F12 = 2 : 1 : 2, which is the original prescription of ERDF. It is not optimized. The serum-free culture medium of the present invention has ERDF as its basal synthetic culture medium. Further, the concentration of inorganic iron was increased within the culture medium. The preferred one is the IES - ERDF having strengthened the concentration of inorganic iron to 40 - 100 $\mu$M per liter of the culture medium or the ES - ERDF having strengthened the concentration of the inorganic iron to 40 - 100 $\mu$M. The protein concentration in this culture medium may be preferably reduced to 5 mg or less of protein per liter of the basal culture medium, or no protein. If such procedure is followed, the protein concentration would be lowered and it becomes easier to refine cell products.

For lowering the protein content, it may be advisable to keep insulin, for example, at about 5 $\mu$g/ml (IES - ERDF having enriched the inorganic iron concentration up to 40 - 100 $\mu$M) in the culture medium.

In case of the non-protein culture medium, it may be advisable to use ,for example, the ES-ERDF having strengthened the inorganic iron concentration up to 40 - 100 $\mu$M/1.

Even if such culture medium is used, it is possible to carry out the successive cultivation of such animal cells as hybridoma, etc. for a prolonged period.

Further, there is no difference with ITES-ERDF in antibody yield.

The cells culturable in the ITES-ERDF are capable of successive culture in the inorganic iron - enriched IES-ERDF and inorganic iron - enriched ES - ERDF. Thus, this inorganic iron - enriched serum-free culture medium is suitable for the culture of mouse hybridoma derived from such parental stocks as SP2, NS1 or X63.

Those which may be used as inorganic iron are iron sulphate, iron chloride, iron nitrate or hydrate thereof.

The present invention comprises providing a novel serum-free culture medium having inorganic iron compound added as a growth factor to the basal culture medium. By using the culture medium of the present invention, it is possible to culture animal cells as efficiently as when serum containing culture medium is used. Further, it is easier to purify products than when the serum culture medium is used.

The present invention is particularly useful when ERDF is used as the basal synthetic culture medium.

Now, mention is made of the abbreviations about the culture medium of the present invention.

"I" represents insulin; "T", transferrin; "E", ethanol amine ; and "S", soduium selenite.

The designation put before ERDF shows that such and such ingredients are contained (for example, ES-ERDF = ERDF containing ethanol amine and selenium.

Further, "R" of the culture medium RDF shows "RPMI 1640", "D" thereof, DMEM; "F", Ham's F12. These show that RDF comprises these culture media.

Figure 1 shows the relationship between the concentration of Iron (II) sulfate and growth promotion when iron (II) sulfate is added to IES - ERDF and hybridoma is cultured.

Figure 2 shows the relationship between the kind of iron salts and growth promotion of hybridoma when it is cultured in the medium having various salts added thereto

Figure 3 shows the growth promotion of the hybridoma when it is cultured in the non-protein culture medium with iron (II) sulfate added.

Figure 4 shows the change in the growth promotion rate when subjected to successive culture in the medium having iron (II) sulfate added thereto.

The present invention is concretely illustrated by way of examples.

Example 1
(1) Cell

There has been used the anti-bovine lactoferrin antibody secretion mouse hybridoma HB8852. (Journal of Dairy Science, Vol 70, pages 752 - 759, 1987).

This hybridoma secrets IgG type antibody. The cells have been subjected to successive culture in ERDF (Kyokuto Seiyaku) containing 10% Fetal Calf Serum (FCS) (Flow Inc).

A tissue culture flask having 25 cm$^2$ of base area, (Corning 25102S) was used for the successive cultivation of cells. The cells were cultured in 5% carbon dioxide /95% air, 90% or higher of humidity, at 37 °C.

(2) Serum-Free Culture Medium of Hybridoma

There have been used, as the basal synthetic culture medium, the ERDF (Kyokuto Seiyaku) containing insulin (5$\mu$g/ml, Nobo Inc., biphasic insulin), ethanolamine (20 $\mu$M, Sigma Inc.) and selenium (sodium selenite, 25 M Sigma Inc. ) (IES), or ethanolamine and selenium (ES), added thereto.

There has been used, as iron preparation, iron (II) sulfate heptahydrate iron (III) chloride , iron (III) nitrate enneahydrate (all of them, Wako Seiyaku).

Before transferring the HB8852 to the serum-free culture medium, hybridoma was subjected to successive culture in the ERDF (ITES-ERDF) containing insulin,transferrin, ethanolamine and selenium for at least one week. Thereafter, the cells have been washed three times with the ERDF containing no ITES. This was for avoiding the influence from the FCS used for the successive culture.

(3) Result
A. Hybridoma Growth Promotion Effect by Iron (II) Sulfate

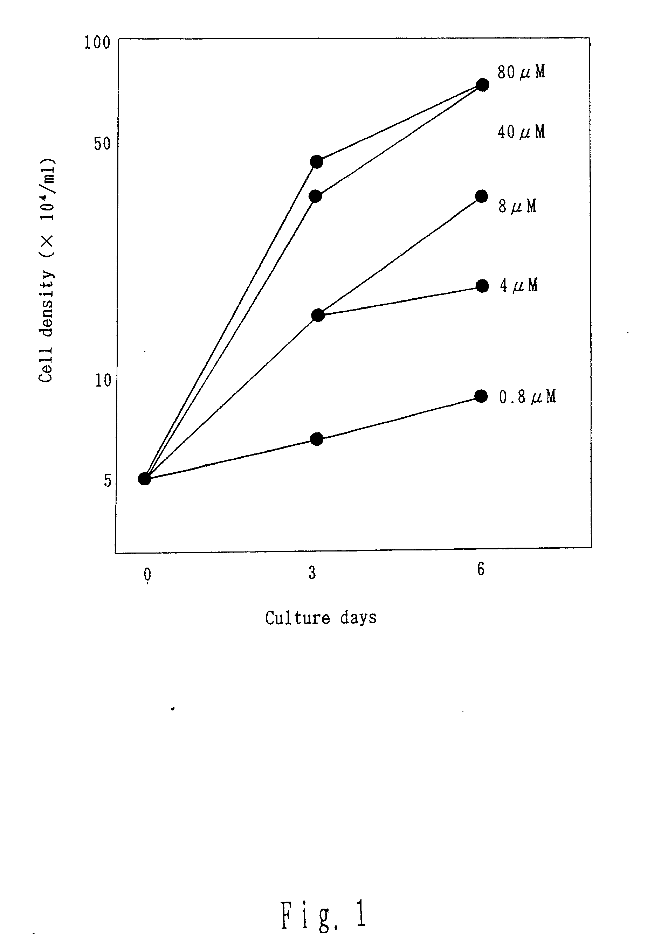

Iron (II) sulfate was added to the IES-ERDF. The hybridoma was cultured. The concentration of iron (II) sulfate originally contained in the ERDF is 0.8 $\mu$M. In this experiment, iron (II) sulfate was added to the IES-ERDF in the concentration of 5 - 100 times (4 - 80 $\mu$M) as much as that of the original concentration thereof. As shown in Figure 1, as the volume of iron (II) sulfate increases, the growth of the hybridoma is promoted.

In the culture medium in which 40 $\mu$M or 80 $\mu$M of iron (II) sulfate is added, the cell density reached 7.2 x $10^5$/ml six days after the culture.

B. Hybridoma Growth Promotion by Four Kinds of Inorganic Iron

In order to find whether the growth of the hybridoma will be influenced by the kind of iron preparations used, hybridoma HB 8852 was cultured on the IES-ERDF having therein respectively 80 $\mu$M of iron (II) sulfate, iron (III) chloride , iron (III) nitrate and potassium ferricyanide [$K_3Fe(CN)_6$].

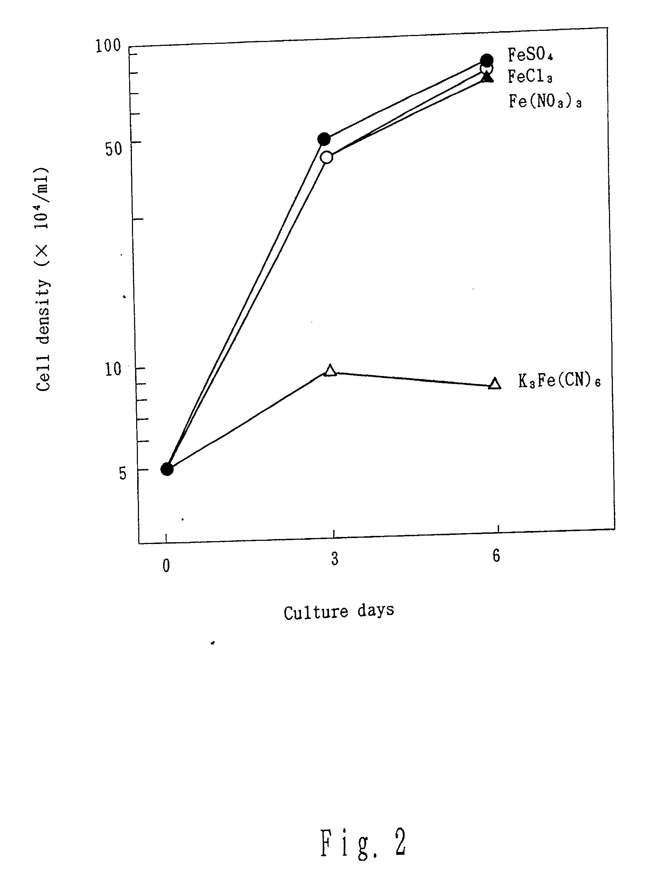

As shown in Figure 2, the cell density of the hybridoma showed the highest value in the culture medium having therein iron (II) sulfate added both three days and six days after it was subjected to the culture.

It reached cell density of 8.2 X $10^5$/ml six days thereafter. Iron (III) chloride and iron (III) nitrate showed the same degree of the growth promotion as that of iron (II) sulfate. They showed the cell density reached six days thereafter respectively 7.8 x $10^5$/ml and 7.3 X $10^5$/ml.

C. Hybridoma Growth Promotion in Protein-Free Culture Medium By iron (II) Sulfate

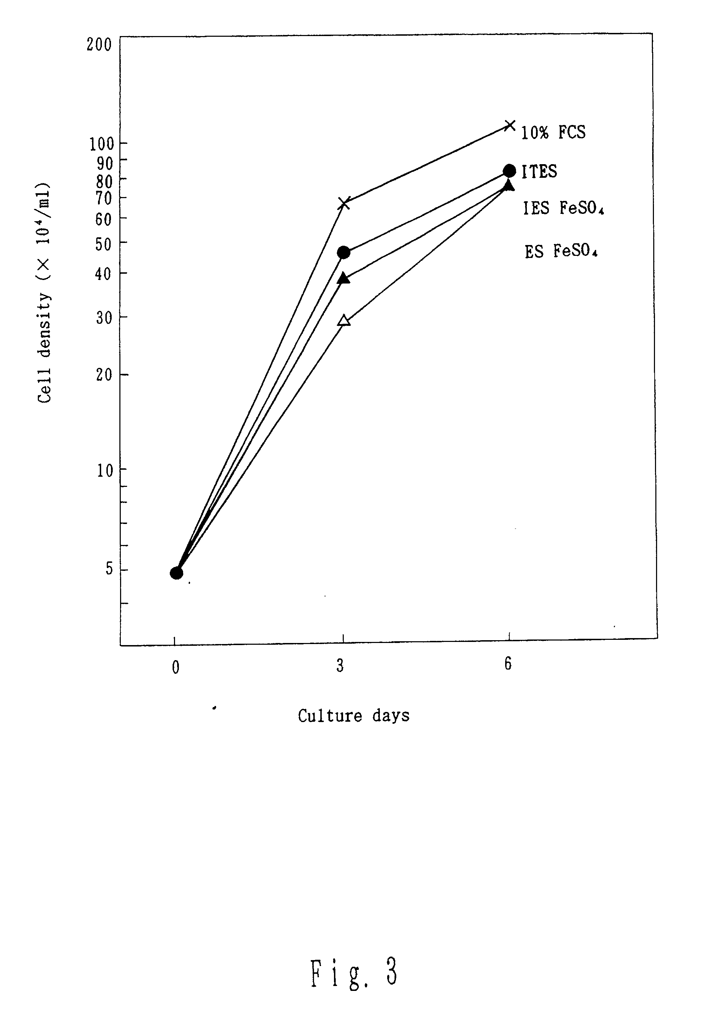

80 $\mu$M of iron (II) sulfate was added to the ES-ERDF. The hybridoma HB8852 was cultured. As shown in Figure 3, the good growth of the hybridoma was shown even in the absolutely non-protein culture medium (ES-FeSO$_4$ ) containing no insulin.

Example 2
(1) Long-Term Cultivation of Hybridoma

The hybridoma was seeded at the density of 1 X $10^5$/ml, in 10 ml of IES-ERDF or ES-ERDF to which 80 $\mu$M of iron (II) sulfate was added. The hybridoma was taken into a tissue culture flask. The cell density was measured every 3 - 4 days thereafter. It was diluted so as to be about 1 X$10^5$/ml in the fresh culture medium and subjected to successive culture for 17 days.

(2) Result

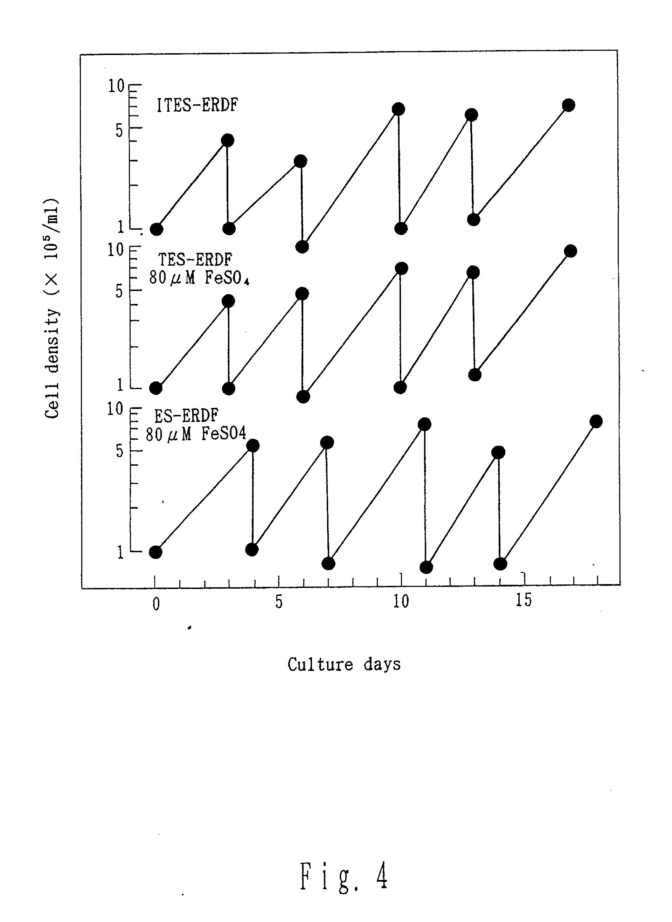

Figure 4 shows the result of the long-term culture of the hybridoma HB 8852 in the IES-ERDF or ES-ERDF containing 80 $\mu$M of iron (II) sulfate.

The growth rate of cells was not reduced during the culture for 17 days.

The mouse hybridoma HB8852 was capable of successive culture in the inorganic iron enriched IES-ERDF containing insulin only as protein or the inorganic iron enriched ES-ERDF containing absolutely no protein in the same way as in the ITES-ERDF containing insulin and transferrin.

Example 3
(1) Measurement of Antibody Secretion Ability

The HB8852 was seeded into a tissue culture flask, at a density of 0. 5 X $10^4$/ml, in the iron (II) sulfate enriched IES-ERDF, ES-ERDF, or the ERDF having therein 10 % FCS added, and cultured for three days.

Then, IgG secreted into the supernatant of the culture was measured by ELISA (Immunochemistry , Vol 8, pages 871-874,1971.)

(2) Result

Antibody Yield by Hybridoma in Inoranic Iron Enriched Serum-Free Culture Medium

The HB8852 was cultured in the iron (II) sulfate enriched IES-ERDF, iron (II) sulfate enriched ES-ERDF or ERDF having therein l0 % FCS added. The density of the antibody (IgG) secreted into the culture three days after the culture was measured by ELISA. Table 2 shows the result. The ERDF containing therein 10 % FCS added was used as the control. The antibody secretion therein was 0.216 ($\mu/10^4$ cells).

On the contrary, the antibody production yield in the iron (II) sulfate enriched IES-ERDF and iron (II) sulfate enriched ES-ERDF has reached twice as much as the yield in the ERDF having therein 10 % FCS added.

## Table 2  Antibody Productivity  Yield By HB8852 in Serum-Free Culture Medium

| culture medium | antibody(IgG) concentration ($\mu g/ml$) | cell density ($\times 10^4$ /ml) | yield per cell ($\mu g/10^4$ per cell) |
|---|---|---|---|
| FeSO$_4$ enriched IES-ERDF | 20.0 | 37.8 | 0.529 |
| FeSO$_4$ enriched ES-ERDF | 15.0 | 27.3 | 0.549 |
| 10% FCS added ERDF | 14.0 | 64.8 | 0.216 |

## Claims

1. A serum-free culture medium containing inorganic iron compound as a growth factor added to a basal synthetic culture medium.

2. The serum-free culture medium according to Claim(1) in which the serum-free culture medium is ERDF.

3. The serum-free culture medium accoring to either Claim(1) or (2) in which the inorganic iron compound is at least one member selected from the group consisting of iron (II) sulfate, iron (III) nitrate and iron (II) chloride.

4. The serum-free culture medium according to either Claim(1), (2) or (3) in which the concentration of inorganic iron compound is in the range of 40$\mu$M to 100 $\mu$M of basal synthetic culture medium.

5. The serum-free culture medium according to either Claim(1), (2), (3) or (4) in which the basal synthetic culture medium is a culture containing, as a growth factor, ethanolamine and/or sodium selenite.

6. The serum-free culture medium according to either Claim(1), (2), (3), (4) or (5) in which the basal synthetic culture medium is a culture containing 5 mg or less of protein per liter of basal synthetic culture medium.

7. The serum-free culture medium according to either Claim(1), (2), (3), (4), (5) or (6) which contains the protein insulin.

8. The serum-free culture medium according to either Claim(1), (2), (3), (4) or (5) in which the basal synthetic culture medium is a culture containing absolutely no protein.

9. The serum-free culture medium according to either Claim(1), (2), (3), (4), (5), (6) or (7) in which the basal synthetic culture medium is a culture containing absolutely no transferrin.

Fig. 1

Fig. 2

Fig. 3

Fig. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | EP-A-0 389 786 (W.R.GRACE & CO.-CONN.)<br>* page 4, lines 14 - 17 * * pages 8 - 14 *<br>- - - | 1-9 | C 12 N 5/00 |
| X | EP-A-0 274 445 (MEDI-CULT A/S)<br>* the whole document *<br>- - - | 1-2,5-9 | |
| X | BIOTECHNOLOGY LETTERS vol. 9, no. 4, 04 April 1987, KEW ENGL. pages 259 - 264; Kovar J. et al: "Iron compounds at high concentrations enable hybridoma growth in a protein free medium"<br>* the whole document *<br>- - - | 1-9 | |
| X | GB-A-2 196 348 (CESKOSLOVENSKA AKADEMIE VED)<br>* the whole document *<br>- - - | 1-9 | |
| X | JOURNAL OF IMMUNOLOGICAL METHODS vol. 116, no. 1, 06 January 1989, HOLLAND pages 65 - 77; Schneider,Y.: "Optimization of hybridoma cell growth and monoclonal antibody secretion...."<br>* the whole document *<br>- - - | 1-3,5-9 | |
| A | BIOLOGICAL ABSTRACT,vol 88,1989,Takazawa Y.et al "High cell density perfusion culture of hybridom & Cytotechnology, vol 1(2), pages 171-178,1988, abstract number 44431<br>* abstract *<br>- - - | 2 | **TECHNICAL FIELDS SEARCHED (Int. Cl.5)**<br><br>C 12 N |
| A | BIOTECHNIQUES vol. 6, no. 1, 1988, NATICK MASS. pages 62 - 67; Wolfe,R. et al: "A new serum free medium for monoclonal antibody production"<br>* page 2, column 1 *<br>- - - - - | 5 | |

The present search report has been drawn up for all claims

| Place of search | Date of completion of search | Examiner |
|---|---|---|
| The Hague | 15 March 91 | FERNANDEZ Y BRANAS F |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document